# EUROPEAN PATENT APPLICATION

(11) **EP 2 015 058 A1**
(43) Date of publication of application: **14.01.2009**
(21) Application number: 08158644.8
(22) Date of filing: 20.06.2008
(51) Int. Cl.: G01N 25/32, G01N 33/22, G01N 27/16

(54) **Wobbe index sensor system**

(30) Priority: 13.07.2007 US 777575
(71) Applicant: GENERAL ELECTRIC COMPANY, Schenectady, NY 12345 (US)
(72) Inventor: Knobloch, Aaron Jay, Mechanicville, NY 12118 (US); Antel, Jr., William Joseph, 85354, Freising (DE); Christoforo, Mark Greyson, Minden, NV 89423-0840 (US); Orenstein, Robert Michael, Atlanta, GA 30309 (US)
(74) Representative: Bedford, Grant Richard

(57) **Abstract**

A sensor (10) for measuring Wobbe index of a fuel is provided. The sensor (10) includes a substrate (12) and a diaphragm layer (16). The diaphragm layer (16) includes a first layer (18) having at least one heating element configured to sense energy content in a fuel, wherein the heating element includes a doped poly-silicon carbide that is disposed on the substrate (12). The diaphragm layer (16) also includes a second layer (22) including an undoped poly-silicon carbide layer configured to prevent oxidation of the first layer (18). The sensor (10) further includes a sensing layer (22) having a catalyst suspended in a support structure. The sensor (10) also includes a cavity formed under the diaphragm layer (16) and is configured to provide thermal isolation of the heating element.

## Description

The invention relates generally to monitoring gas turbine engine systems and, and more particularly, to a system for measuring a lower heating value and Wobbe index of a fuel.

There is an increasing demand for gas-fired combustion systems that can be readily operated on liquefied natural gas (LNG), blends of pipeline natural gas and LNG, and a variety of low BTU (British thermal unit) fuels. Accordingly, there is a demand for systems that can determine a lower heating value or a Wobbe index of the fuels to ensure an engine performance is matched to characteristics of the fuel.

One of the commonly used systems for determining Wobbe index includes a gas chromatograph (GC) system. The GC system includes a glass capillary system to separate fuel constituents and a thermal conductivity detector or a flame ionization detector (FID) to quantitatively identify the constituents. However, the GC system provides measurements that are not continuous but over about 10 minute intervals. Further, the system is relatively expensive and difficult to operate.

Another commonly used system includes a calorimeter system to determine fuel quality, wherein the energy of fuel combustion is directly measured. These systems are sensitive to both the fuel composition and the ambient temperature, creating large errors when the ambient temperature varies.

One method for making accurate measurement of gas concentrations is using microfabricated hotplates. The microhotplates are typically coated with a gas sensitive coating and calorimetric measurements of a reaction of the coating with the gas of interest is made. For many applications it is advantageous to operate the microhotplates at an elevated temperature to enable reaction or adsorption of the gas to the gas sensitive coating. Typically, the microhotplates are composed of metal heater layers such as platinum (Pt) on top of a ceramic layer such as silicon nitride. Since the microhotplates operate at elevated temperature, the temperature coefficient of expansion of dissimilar materials that compose the microhotplates cause high stresses that can lead to failure of the microhotplates. In addition, devices may be fragile in construction due to high residual stresses of the microhotplates generated during fabrication.

Therefore, a need exists for an improved system for determining fuel quality that may address one or more of the problems set forth above.

In accordance with one aspect of the invention, a sensor for measuring Wobbe index of a fuel is provided. The sensor includes a substrate and a diaphragm layer. The diaphragm layer includes a first layer having at least one heating element configured to sense energy content in a fuel, wherein the heating element includes a doped poly-silicon carbide that is disposed on the substrate. The diaphragm layer also includes a second layer including an undoped poly-silicon carbide layer configured to prevent oxidation of the first layer. The sensor further includes a sensing layer disposed on the diaphragm layer having a catalyst. The sensor also includes a cavity formed by the removal of the substrate under the diaphragm layer. This provides thermal isolation of the heating element from the substrate.

In accordance with another aspect of the invention, a system for measuring Wobbe index of a fuel is provided. The system includes a flow control device configured to control at least one of a flow rate of air and a flow rate of a fuel for providing a combustible air-fuel mixture. The system also includes a first sensor in flow communication with the flow control device. The first sensor includes a substrate and a diaphragm layer. The diaphragm layer includes a first layer having at least one heating element configured to sense energy content in a fuel, wherein the heating element includes a doped poly-silicon carbide layer that is disposed on the substrate. The diaphragm layer also includes a second layer including an undoped poly-silicon carbide layer configured to prevent oxidation of the first layer. The first sensor also includes a first cavity formed under the diaphragm layer, which provides thermal isolation of the at least one heating element from the substrate. The system also includes a second sensor in flow communication with the flow control device. The second sensor includes a substrate and a diaphragm layer. The diaphragm layer includes a first layer having at least one heating element configured to sense energy content in a fuel, wherein the heating element includes a doped poly-silicon carbide layer that is disposed on the substrate. The diaphragm layer also includes a second layer including an undoped poly-silicon carbide layer configured to prevent oxidation of the first layer. The second sensor further includes a sensing layer disposed on the diaphragm layer having a catalyst. The second sensor also includes a second cavity formed under the diaphragm layer, which provides thermal isolation of the at least one heating element from the substrate. The system further includes a sensor exhaust in flow communication with the sensor and configured to measure a volumetric flow rate of combustion products.

Various features, aspects, and advantages of the present invention will become better understood when the following detailed description is read with reference to the accompanying drawings in which like characters represent like parts throughout the drawings, wherein:
FIG. 1 is a cross-sectional view of a sensor used to measure Wobbe index of a fuel in accordance with an embodiment of the invention;
FIG. 2 is a top view of the sensor in FIG. 1;
FIG. 3 is a schematic illustration of a system for measuring a Wobbe index for a fuel using the sensor in FIG. 1 in accordance with an embodiment of the invention; and
FIG. 4 is a graphical illustration of resistance of two sample Wobbe index sensors as a function of temperature.

As discussed in detail below, one embodiment of the present invention include a sensor for measuring Wobbe index and lower heating value (LHV) of a fuel. Although the present discussion focuses on a sensor for a gas turbine engine in an industrial environment, the present system is not limited to gas turbines, but is also applicable to other applications such as measurement of fuel quality in a jet engine or fuel composition measurement in a pipeline. Further, the principles and teachings set forth herein are applicable to a variety of gaseous and liquid combustible fuels, such as but not limited to, natural gas, gasoline, kerosene, diesel fuel and jet fuel.

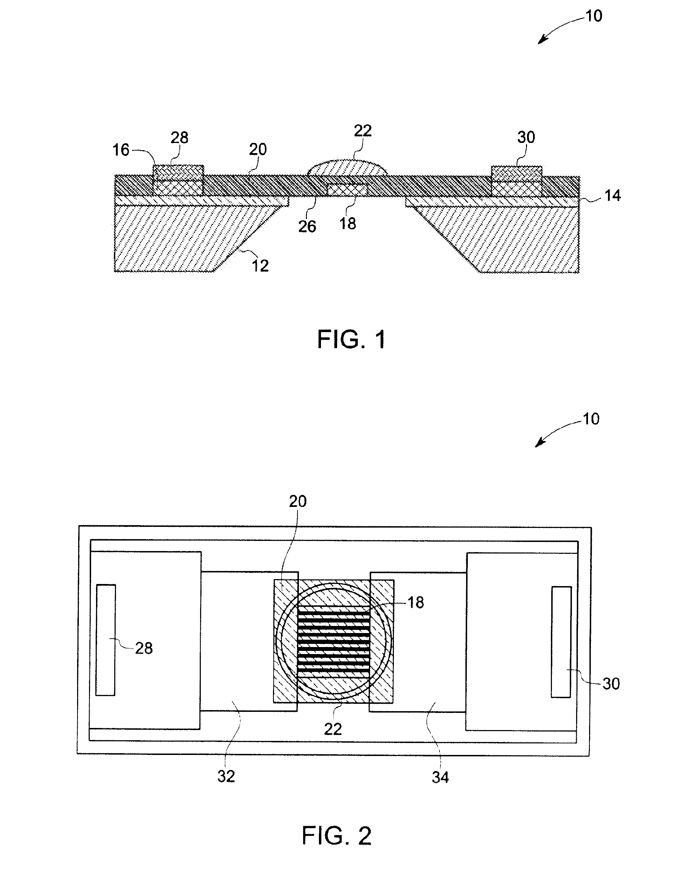

Turning to the drawings, FIG. 1 is a cross-sectional view of a sensor 10 for measuring Wobbe index and LHV of fuel. The sensor 10 includes a substrate 12. In a particular embodiment, the substrate 12 includes a silicon substrate. An insulating layer 14 is disposed on the substrate 12. In an example, the insulating layer 14 includes a silicon oxide layer. Further, a diaphragm layer 16 is disposed on the insulating layer 14. The diaphragm layer 16 includes a first layer or heating element 18 and is typically made of doped poly-silicon carbide. In a non-limiting example, the dopant includes at least one of a n-type or p-type dopant such as, but not limited to, nitrogen, boron, aluminum, a group I, II, III, or V element. In an exemplary embodiment, the heating element includes multiple patterned resistors either in series or parallel. A passivation layer 20, also referred to as a second layer, is made of undoped poly-silicon carbide and is coated on the first layer 18 to protect against oxidation, consequently enabling a constant resistance during operation. Furthermore, silicon carbide is known to be resistant to oxidation and chemically resistant to combustion products that reduce the risk of diaphragm cracking.

A sensing layer 22 includes a catalyst suspended in a support structure so as to initiate combustion of the air-fuel mixture at relatively low temperatures. In a presently contemplated embodiment, the sensing layer 22 includes a layer of platinum suspended in alumina. Some non-limiting examples of the catalyst 22 include a noble metal, noble metals with additives such as copper, semiconductor oxides and hexaaluminate materials. Examples of the supporting structure apart from alumina include hexaaluminates, zirconia, ceria, titania and hydrous metal oxides. A cavity 26 is formed under the diaphragm layer 16 and provides thermal isolation of the heating element 18 from the ambient environment. Contact pads 28 and 30 including a metal provide electrical connection to the heating element 18.

FIG. 2 is a top view of the sensor 10 of FIG.1. The sensor 10 includes multiple heating elements 18 and the passivation layer 20. The heating elements 18 and the passivation layer 20 are typically made of polycrystalline silicon carbide. The diaphragm layer 16, as referred to in FIG. 1, including the heating elements 18 and the passivation layer 20 may also be termed a micro-hotplate. The heating elements 18 are configured to heat the micro-hotplate on application of an electrical current. In the illustrated embodiment, the heating elements 18 include a doped silicon carbide material that can sustain substantially high temperatures and harsh environments. The sensing layer 22 including a catalyst with a supporting structure is coated on the diaphragm layer 16 to initiate combustion. The diaphragm layer 16 also includes contact pads 28 and 30 for facilitating the electrical connections for the sensor 10. In the presently contemplated embodiment, the contact pads 28 and 30 include doped poly-silicon carbide and Ni/Au layers. In certain embodiments, the contact pads 28 and 30 may also include other suitable metals. Further, a contact pad material may be deposited on the silicon carbide contact pads 28 and 30. Examples of such materials include titanium, tungsten, gold, nickel and combinations thereof. Elements 32 and 34 including doped poly silicon carbide provide for electrical connection between the contact pads 28 and 30 and the heating elements 18.

Silicon carbide is used advantageously in the heating elements 18 and the passivation layer 20 of the diaphragm layer resulting in a robust, reliable design of the sensor 10 desirable for operation in harsh environments. It avoids usage of materials with different coefficients of thermal expansions such as, but not limited to, ceramics and metals. Further, fabrication of the diaphragm layer 16 becomes reliable and repeatable.

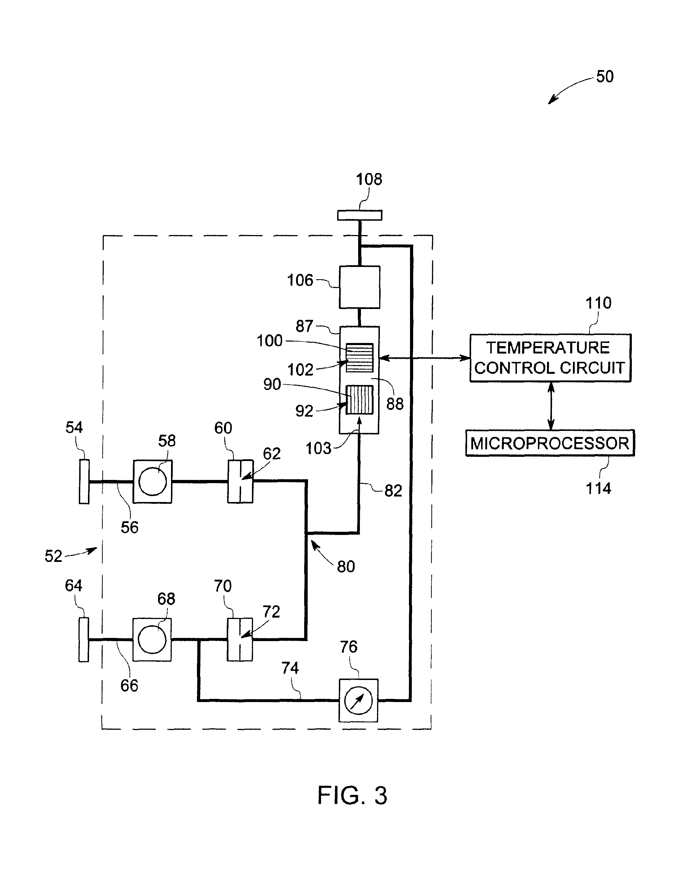

FIG. 3 is a block diagram representation of a system 50 including the sensor 10 to measure a LHV and a Wobbe index of a fuel. The system 50 includes a flow control device 52 that controls a flow rate of a mixture of combustible fuel and air hereinafter referred to as "air-fuel mixture." In one embodiment, proportions of air to fuel are fixed such that an equivalence ratio, is less than 1 as in the case of a lean flow mixture. Flow control device 52 includes at least one air inlet 54 that directs a supply of air through an air supply line 56. A pressure regulator 58 regulates the pressure of air through the flow control device 52. An orifice plate 60 defines an orifice 62 in flow communication with the pressure regulator 58. A backing pressure through the orifice 62 is adjusted using the pressure regulator 58 such that the air flow through the orifice 62 is choked. Velocity of the air flowing through the orifice 52 will be substantially constant with upstream pressure of the orifice 52 sufficiently higher than downstream pressure. Such a flow is referred to as a "choked" flow. By maintaining a choked air flow condition, the mass flow of the air is kept substantially stable when compared to an unchoked air flow.

Similarly, a supply of fuel, such as natural gas, is directed through at least one fuel inlet 64 and through a fuel supply line 66. A pressure regulator 68 regulates the pressure of fuel through the flow control device 52. An orifice plate 70 forms an orifice 72 in flow communication with a pressure regulator 68. A backing pressure through orifice 72 is adjusted using the pressure regulator 68 such that the fuel flow through the orifice 72 is choked. With the pressure upstream of the orifice 72 sufficiently higher than the pressure downstream, velocity of the fuel flowing through the orifice 72 will be substantially constant, e.g., a "choked" flow. By maintaining a choked fuel flow condition, the mass flow of the fuel is kept substantially stable when compared to an unchoked fuel flow. It is possible to have changes in mass flow if the density of the fuel changes. However, the variation in mass flow is easily calculated given a density of the fuel and the choked flow.

The flow control device 52 and, more specifically, the pressure regulators 58, 68 are configured to balance the mass flow of fuel with the mass flow of air to achieve a desired air to fuel ratio. Additionally, the flow control device 52 is suitable to control the flow of high temperature fuels having a temperature up to at least about 200°C, and filters the fuels of contaminants, such as particulates and tars.

In a particular embodiment, a bypass 74 is in flow communication with fuel supply line 66. The bypass 74 is operatively controlled by a variable needle valve 76 to provide or allow an increased fuel flowrate through the bypass 74 to supply a suitable amount of fuel to a sensor positioned downstream, as desired. For example, the orifice 72 may control the fuel flow through the fuel supply line 66 down to a small value such that a sensor response time is undesirably increased due to the overall distance between the fuel inlet 64 and the sensor positioned downstream. To decrease the sensor response time, the variable needle valve 76 is activated to open and provide an increased fuel flow through the bypass 74 to supply a suitable amount of fuel to the sensor.

The supply of air and the supply of fuel are combined and mixed at a piping junction 80. A main air-fuel supply line 82 is in flow communication with each of the air supply line 56 and the fuel supply line 66 at the piping junction 80. In an exemplary embodiment, a percentage of air and a percentage of a fuel for the air-fuel mixture are selected such that the air-fuel mixture is combustible. The main air-fuel supply line 82 directs the air-fuel mixture through a sensor fixture 10 in flow communication with the main air-fuel supply line 82. In one embodiment, the sensor 10 includes an enclosure 87 that defines a chamber 88 therein.

The system 50 includes a first or reference micro-hotplate or sensor 90 and a second or catalyst micro-hotplate or sensor 100 positioned with respect to the reference microhotplate 90. The first sensor 90 and the second sensor 100 refer to the sensor 10 in FIG. 1. The reference micro-hotplate 90 and the catalyst micro-hotplate 100 are positioned within the chamber 88. In one embodiment, the reference micro-hotplate 90 is aligned in series with the catalyst micro-hotplate 100 with respect to a direction of air-fuel mixture flow through chamber 88, as shown by directional arrow 103. In an alternative embodiment, the reference micro-hotplate 90 is aligned in parallel with catalyst micro-hotplate 100 with respect to a direction of air-fuel mixture flow through chamber 88. It will be appreciated by those skilled in the art that the two micro-hotplates namely, the reference micro-hotplate 90 and the catalyst micro-hotplate 100, are shown for simplicity. However, the system 50 may include multiple reference micro-hotplates 90 and multiple catalyst micro-hotplates 100 in a series or a parallel combination to increase the combustion conversion efficiency.

In a particular embodiment, the reference microhotplate 90 includes a silicon carbide membrane on a silicon substrate. As the air-fuel mixture flows across a flow surface 92 of the reference micro-hotplate 90, heat from the reference micro-hotplate 90 is transferred to the air-fuel mixture. Since the reference micro-hotplate 90 is identical to the sensing micro-hotplate 100 but no reaction with the gas takes place, the convective and conductive losses from the micro-hotplate can be measured.

The air-fuel mixture further flows across the catalyst micro-hotplate 100. In a particular embodiment, the catalyst micro-hotplate 100 includes a silicon carbide membrane on a silicon substrate. At least a portion of the catalyst micro-hotplate 100 is coated with a catalyst in a supporting structure. The catalyst is supported in high-temperature-stable, high-surface-area materials. In an example, the catalyst is platinum supported in alumina. Some non-limiting examples of the catalyst include noble metals such as palladium. Other non-limiting examples of a supporting structure include hexaaluminates, zirconia, ceria, titania or hydrous metal oxides such as, but not limited to, hydrous titanium oxide (HTO), silica-doped hydrous titanium oxide (HTO:Si), and silica-doped hydrous zirconium oxide (HZO:Si). The catalysts with a supporting structure have good stability and reactivity. As the air-fuel mixture flows across the surface of the catalyst micro-hotplate 100, the air-fuel mixture is initiated to combust as a result of contact with a coating of the catalyst, and the heat of combustion reduces the power requirement for the catalyst microhotplate 100. The catalyst micro-hotplate 100 is configured to measure flow and thermal variation of the system when exposed to gas flow. The measurement increases accuracy of the system when compared to the reference micro-hotplate 90. The reference micro-hotplate 90 includes a non-reactive film coating such as, but not limited to, alumina in absence of a catalyst.

The supported catalyst can be deposited on a flow surface 102 of the catalyst micro-hotplate 100 that is exposed to the flow of the air-fuel mixture. Reliable deposition of catalysts is desirable in order to achieve consistent performance. The catalysts are deposited onto flow surface 102 of the catalyst micro-hotplate 100 using any suitable deposition process known in the art. Combustion products resulting from the combustion of the air-fuel mixture within the sensor 10 are directed through a sensor exhaust 106 before being released into the atmosphere through an exhaust outlet 108.

A temperature control system 110 is configured to maintain an optimal temperature for operation and is in communication with the sensor 10. The temperature control system 110 maintains the reference micro-hotplate 90 and the catalyst micro-hotplate 100 at a constant temperature. Further, the temperature control system 110 facilitates active control of the reference micro-hotplate 90 and the catalyst micro-hotplate 100 by varying power into the reference micro-hotplate 90 and the catalyst micro-hotplate 100 in order to maintain a fixed resistance, and consequently a desirable temperature. In a particular embodiment, the temperature control system 110 measures power required to maintain a constant temperature. In a particular embodiment, heat from the reference micro-hotplate 90 is transferred to the air-fuel mixture and by monitoring a change in power supplied to the reference micro-hotplate 90 required to maintain a constant temperature, a convective or a conductive power loss is measured. In another embodiment, when external heating from combustion attempts to increase temperature of the flow surface 102, the temperature control system 110 decreases power to compensate and consequently maintain the catalyst micro-hotplate 100 at a constant temperature.

A microprocessor 114 interfaced with the temperature control system 110 is configured to monitor and record measurements taken within the sensor 10. Further, the LHV and Wobbe index of the fuel is computed by the microprocessor 114. In a particular embodiment, the sensor exhaust 106 may be coupled to the microprocessor 114 to provide feedback of mass flow through the exhaust outlet 108. In a particular embodiment, the temperature is maintained at about 400°C. An overall change in power supplied to the reference micro-hotplate 90 and the catalyst micro-hotplate 100 is directly related to the LHV. Further, a time response of the reference micro-hotplate 90 and the catalyst micro-hotplate 100 is of the order of milliseconds resulting in a real time measurement of the LHV.

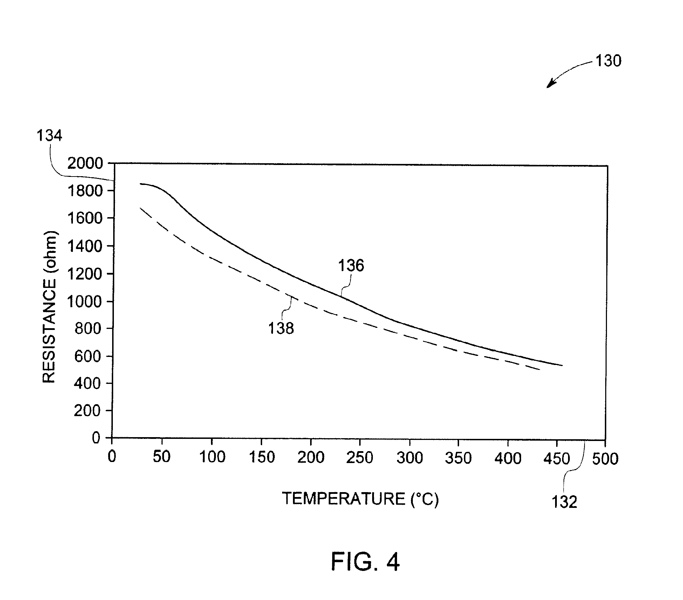

FIG. 4 is a graphical illustration 130 of a measured resistance as a function of temperature for two sample Wobbe index sensors. The X-axis 132 represents temperature measured in degrees Celsius and the Y-axis 134 represents resistance measured in ohm. The temperature is measured from the sensors via infrared pyrometry. The resistance is determined via Ohm's law given by V=I*R, wherein V is voltage, I is current and R is the resistance, by measuring the voltage and the current supplied to the sensors. The resistance decreases exponentially with temperature as illustrated by curves 136 and 138. Curve 136 represents resistance measurement for a first sensor and the curve 138 represents resistance measurement for a second sensor. A least squares fit to an exponential function is performed on the curves 136 and 138 to obtain a functional relationship between resistance and temperature. The relationship is used to set the micro-hotplate to a desired temperature during operation. Those skilled in the art will recognize that other functional relationships can be used, for example *Steinhart equation.* The curves 136 and 138 differ from one another due to variations in manufacturing. The variations include but are not limited to small differences in diaphragm thickness, doping levels, and resistor track widths.

The various embodiments of a Wobbe index sensor and a system for detecting Wobbe index and LHV described above thus provide a way to achieve, efficient and accurate measurement of energy content in a fuel. These systems also allow for highly efficient combustion systems due to an improved and real time sensing technique.

Of course, it is to be understood that not necessarily all such objects or advantages described above may be achieved in accordance with any particular embodiment. Thus, for example, those skilled in the art will recognize that the systems and techniques described herein may be embodied or carried out in a manner that achieves or optimizes one advantage or group of advantages as taught herein without necessarily achieving other objects or advantages as may be taught or suggested herein.

While only certain features of the invention have been illustrated and described herein, many modifications and changes will occur to those skilled in the art. It is, therefore, to be understood that the appended claims are intended to cover all such modifications and changes as fall within the true spirit of the invention.

## Claims

1. A sensor (10) for measuring Wobbe index of a fuel comprising:
a substrate (12);
a diaphragm layer (16) comprising:
a first layer (18) comprising at least one heating element configured to sense energy content in a fuel, the heating element comprising a doped poly-silicon carbide that is disposed on the substrate; and
a second layer (20) comprising an undoped poly-silicon carbide layer configured to prevent oxidation of the first layer (18);
a sensing layer (22) comprising a catalyst and disposed on the diaphragm layer (16); and
a cavity formed under the diaphragm layer (16) to provide thermal isolation of the heating element.

2. The sensor (10) of claim 1, further comprising an insulating layer (14) disposed on the substrate (12).

3. The sensor (10) of any preceding claim, wherein the catalyst comprises a layer of metal suspended in a support structure.

4. The sensor (10) of any preceding claim, the sensor (10) configured to sense a temperature based upon a measured resistivity of the heating elements.

5. A system (50) for measuring Wobbe index of a fuel comprising:
a flow control device (52) configured to control at least one of a flow rate of air and a flow rate of a fuel for providing a combustible air-fuel mixture;
a first sensor (90) in flow communication with said flow control device (52), said first sensor (90) comprising:
a first substrate (12);
a first diaphragm layer (16) comprising:
a first layer (18) comprising at least one heating element configured to sense energy content in the fuel, the at least one heating element comprising a doped poly-silicon carbide layer that is disposed on the substrate; and
a second layer (20) comprising an undoped poly-silicon carbide layer configured to prevent oxidation of the first layer (18); and
a first cavity formed under the first diaphragm layer (16) and configured to provide thermal isolation of the t least one heating element;
a second sensor (100) in flow communication with said flow control device, said second sensor (100) comprising:
a second substrate (12);
a second diaphragm layer (16) comprising:
a first layer (18) comprising a plurality of heating elements configured to sense energy content in the fuel, the plurality of heating elements comprising a doped poly-silicon carbide that is disposed on the second substrate (12);
a second layer (20) comprising an undoped poly-silicon carbide layer configured to prevent oxidation of the first layer;
a sensing layer (22) comprising a catalyst and disposed on the second diaphragm layer (16); and
a second cavity formed under the second diaphragm layer (16) configured to provide thermal isolation of the heating elements; and
a sensor exhaust (106) in flow communication with said sensor (10) and configured to measure a volumetric flow rate of combustion products.

6. The system (50) of claim 5, further comprising a temperature control system (110) in control communication with each of said diaphragm layer (16) and said sensing layer (22), said temperature control system (110) configured to maintain said diaphragm layer (16) and said sensing layer (22) at a constant temperature.

7. The system (50) of claim 5 or claim 6, wherein the first sensor (90) and the second sensor (100) comprise a first insulating layer (14) and a second insulating layer disposed on the first substrate (10) and the second substrate respectively.

8. The system (50) of any one of claims 5 to 7, wherein the catalyst comprises a layer of metal suspended in a support structure.

9. The system (50) of any one of claims 5 to 8, the first sensor (90) and the second sensor (100) configured to sense a temperature based upon a measured resistivity of the heating elements.

10. The system (50) of any one of claims 5 to 9, wherein the fuel comprises a gaseous or a liquid fuel system.
